(19) Europäisches Patentamt
European Patent Office
Office européen des brevets

(11) Veröffentlichungsnummer: **0 163 895**
**B1**

(12) **EUROPÄISCHE PATENTSCHRIFT**

(45) Veröffentlichungstag der Patentschrift: **12.04.89**

(51) Int. Cl.⁴: **C 07 D 249/08**

(21) Anmeldenummer: **85104860.3**

(22) Anmeldetag: **22.04.85**

(54) **Verfahren zur diastereoselektiven Reduktion von -Triazolyl ketonen zu ss-Triazolylcarbinolen.**

(30) Priorität: **04.05.84 DE 3416444**

(43) Veröffentlichungstag der Anmeldung:
**11.12.85 Patentblatt 85/50**

(45) Bekanntmachung des Hinweises auf die Patenterteilung:
**12.04.89 Patentblatt 89/15**

(84) Benannte Vertragsstaaten:
**AT BE CH DE FR GB IT LI NL**

(56) Entgegenhaltungen:
**DE-A- 2 737 489**
**DE-A- 3 019 049**
**DE-B- 2 324 010**
**GB-A- 2 041 927**

(73) Patentinhaber: **BASF Aktiengesellschaft, Carl-Bosch-Strasse 38, D-6700 Ludwigshafen (DE)**

(72) Erfinder: **Thieme, Peter C., Dr., Dr.-Hans-Hoffmann-Strasse 5, D-6706 Wachenheim (DE)**
Erfinder: **Sauter, Hubert, Dr., Neckarpromenade 20, D-6800 Mannheim 1 (DE)**
Erfinder: **Reissenweber, Gernot, Dr., Drosselstrasse 15, D-6737 Boehl-Iggelheim (DE)**

**EP 0 163 895 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur diastereoselektiven Reduktion von α-Triazolylketonen zu β-Triazolylcarbinolen.

Es ist bekannt, dass die Diastereomeren von Triazolylalkoholen gegen bestimmte phytopathogene Pilze unterschiedlich stark wirken. Eine hohe Wirksamkeit besitzt z. B. nur das Diastereomer, dessen beide Enantiomere die sterische Anordnung Ia aufweisen.

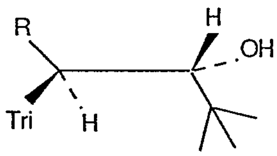 bzw. 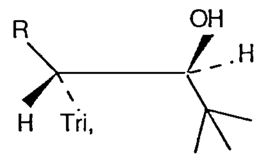 (Ia)

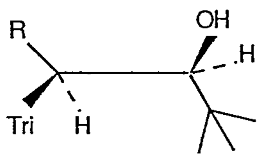 bzw. 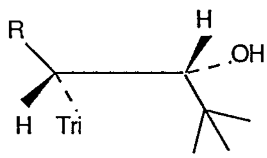 (Ib)

Tri = 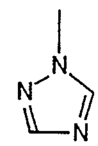

Im Falle von R = 4-Chlorphenoxy entspricht das Enantiomerengemisch Ia dem fungizid wirksameren R*,S*-Diastereomeren des Triadimenols (W. Krämer, K.H. Büchel, W. Draber, 5th International Congress of Pesticide Chemistry, IUPAC, Kyoto 1982 [Abstr. II s–3]).

Zur Erläuterung sei vermerkt, dass Verbindungen mit zwei verschiedenen chiralen Kohlenstoffatomen vier Stereoisomere bilden, deren Konfiguration nach dem Cahn-Ingold-Prelog-System bezeichnet werden kann (siehe z. B. D. Seebach und V. Prelog, Angew. Chem. 94, 696 (1982) und dort angegebene Literatur). Hierbei stellen zwei Enantiomere der Konfigurationen R,R und S,S ein Diastereomer mit der Bezeichnung R*,R* dar. Das andere Diastereomer mit der Bezeichung R*,S* setzt sich aus den beiden Enantiomeren mit den Konfigurationen R,S und S,R zusammen.

In der DE-OS 3 019 049 wurden Beispiele für Verbindungen der oben angegebenen Formel I (z. B. mit R = 4-Phenoxybutyl) aufgeführt, die aus den Diastereomerengemischen R*,R* und R*,S*, und zwar – wegen der Herstellung aus entsprechenden Ketonen mit Hilfe von Natriumboranat (Natriumborhydrid) – überwiegend aus R*,R*-Diastereomeren bestehen müssen. Die Diastereomeren der Konfiguration R*,R* weisen eine erheblich geringere fungizide Wirkung auf als die mit der Konfiguration R*,S*.

Aufgabe der Erfingung ist die Schaffung eines Verfahrens, das gezielt das wirksamere Diastereomere herzustellen erlaubt.

Es wurde nun gefunden, dass die Umsetzung von entsprechenden Ketonen mit einem Ketone zu Alkoholen reduzierenden komplexen Hydrid überwiegend das gewünschte R*,S*-Isomere liefert, wenn man die Umsetzung in einem, bevorzugt unpolaren, Lösungsmittel in Gegenwart einer Lewis-Säure wie Zinkhalogenid oder Titanhalogenid (beispielsweise Titantetrachlorid oder Alkoxytitanchlorid) vornimmt.

Gegenstand der Erfingung ist daher ein Verfahren zur diastereoselektiven Herstellung von β-Triazolylcarbinolen

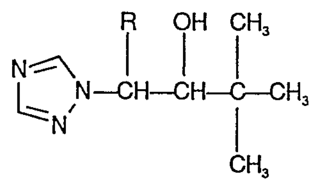 (I)

wobei R

a) Phenoxy, gegebenenfalls substituiert durch

b) $C_1$–$C_{10}$-Alkyl, gegebenenfalls ein- oder mehrfach substituiert durch gleiche oder verschiedene Reste aus den Gruppen Alkoxy, Alkyl, Aryl, Aryloxy, die ihrerseits wiederum durch Halogen, $CF_3$, Alkyl oder Alkoxy substituiert sein können, bedeutet, durch diastereoselektive Reduktion eines entsprechenden α-Triazolylketons mit einem komplexen, Ketone zu Alkoholen reduzierenden Metallhydrid in Gegenwart einer Lewis-Säure, wobei man die Umsetzung in einem aprotischen, vorzugsweise unpolaren Lösungsmittel vornimmt. Man kann jedoch auch in Ethern, z. B. Diethylether oder Polyethern, wie z. B. Dimethoxyethan, arbeiten.

Als Lewis-Säure verwendet man z. B. Zinkchlorid, Zinntetrachlorid, Titantetrachlorid, Zinkbromid, Magnesiumchlorid, Bortrifluorid, Titantetrabromid oder ein Alkoxytitanchlorid der allgemeinen Formel $(R'O)_oTiCl_p$ mit o, p = 1, 3; 2, 2 und 3, 1 in der R′ niederes Alkyl, bevorzugt Isopropyl, bedeutet. Die Lewis-Säuren werden bevorzugt in gleicher molarer Menge wie das Keton eingesetzt.

Das Reduktionsmittel wird zweckmässig in Form eines in unpolaren Lösungsmitteln löslichen Salzes, beispielsweise als Tetra-n-butylammoniumboranat, eingesetzt. Auch andere Boranate wie Tetramethylammonium-, Tetraethylammonium-, Trimethylbenzoylammoniumboranat und entsprechende Aluminiumverbindungen können verwendet werden. Verwendet man Lithium-, Natrium- oder Kaliumboranat, so muss wegen der Schwerlöslichkeit dieser Boranate in unpolaren Lösungsmitteln durch geeignete Hilfsmittel dafür gesorgt werden, dass das Boranat in die organische Phase gelangt. Bei Verwendung von Lithiumborhydrid kann die Beimischung von Ethern wie Diethylether, Methyl-t-butylether oder Tetrahydrofuran die Löslichkeit verbessern. Man kann jedoch z. B. auch mit Natriumborhydrid in Polyethern wie Dimethoxyethan arbeiten.

Als Lösungsmittel kommen bevorzugt halogenierte Kohlenwasserstoffe wie beispielsweise Chloroform, Methylenchlorid, Dichlorethan, aber auch Toluol und Gemische davon in Frage, ferner Ether wie Diethylether, Tetrahydrofuran oder Dimethoxyethan.

Als Beispiele für den Substituenten R seien die folgenden Reste genannt:

Phenoxy
4-Chlorphenoxy
2,4-Dichlorphenoxy
4-Bromphenoxy
2-Chlorphenoxy
2-Fluorphenoxy
4-Fluorphenoxy
3-Trifluormethyl-phenoxy
2,4-Dimethylphenoxy
2,4,6-Trichlorphenoxy
4-Methylphenoxy
2-Methoxyphenoxy
4-Phenylphenoxy
3-Phenylphenoxy
3-Phenoxyphenoxy
4-Phenoxyphenoxy
Benzyl
4-Chlorbenzyl
2,4-Dichlorbenzyl
2-Fluorbenzyl
4-Fluorbenzyl
2-Chlorbenzyl
2-Brombenzyl
2,4-Dimethylbenzyl
4-Methoxybenzyl
2-Methyl-3-phenyl-propyl(1)
3-Phenylpropyl
4-Phenylbenzyl
4-Phenoxybenzyl
2-Phenylethyl
2-Phenoxyethyl
3-Phenoxypropyl
4-Phenoxybutyl
2-(2-Fluorphenoxy)-ethyl
4-(2-Fluorphenoxy)-butyl
2-(4-tert.-Butylphenoxy)-ethyl
2,2,2-Trimethylethyl
n-Decyl
n-Octyl
2-Ethoxyethyl
4-(3-Trifluormethylphenoxy)-butyl
4-(3-Chlorphenoxy)-butyl

Nach dem erfindungsgemässen Verfahren verfährt man so, dass das Keton (II) in etwa 8 bis 15 Gewichtsteilen Lösungsmittel gelöst und unter Kühlung mit der entsprechenden Menge (z. B. 0,3 bis 1,2 Moläquivalent, bevorzugt etwa 0,95 bis 1,05) an z. B. Titantetrachlorid, Zinkchlorid oder einer anderen der oben genannten Lewis-Säuren versetzt wird. Anschliessend wird bei −30°C bis Raumtemperatur, bevorzugt um 0°C, eine Lösung oder Suspension des komplexen Hydrids im entsprechenden Lösungsmittel zugesetzt oder das Hydrid fest zugegeben. Man verwendet einen z. B. 0,1- bis 2fachen Überschuss über die stöchiometrisch benötigte Menge. Als komplexe Hydride kommen alle komplexen Metallhydride in Frage, die Carbonylfunktionen zu Alkoholen reduzieren, beispielsweise $NaBH_4$, $LiBH_4$, $R^4NBH_4$ mit R = H, $C_1$- bis $C_6$-Alkyl, Aralkyl, $LiAlH_4$, Diisobutylaluminiumhydrid (Dibal-4). Bei Verwendung von Lithiumalanat kann dieses auch in Form fester Tabletten zugesetzt werden.

Man lässt auf Raumtemperatur kommen und rührt nach, bis nach dünnschichtchromatographischer Kontrolle das Keton aufgebraucht ist. Das ist je nach verwendetem Reduktionsmittel in bis zu 60 Stunden der Fall. Anschliessend wird wiederum auf 0°C abgekühlt, mit Wasser und verdünnter Salzsäure oder mit wässriger Base, z. B. Natriumhydroxid, versetzt und wie üblich aufgearbeitet. Das Rohprodukt, das in Ausbeuten von i. a. über 50% bis über 90% anfällt, enthält überwiegend bis ausschliesslich das Diastereomere der Konfiguration R*,S*. Die Konfiguration kann durch Analyse des NMR-Spektrums bestimmt werden.

Man hat damit ein Verfahren an der Hand, das unter milden Bedingungen und mit sehr guter Ausbeute praktisch ausschliesslich das gewünschte Diastereomere R*,S* liefert. Gegenüber dem in der DE-OS 3 019 049 beschriebenen Verfahren besitzt das erfindungsgemässe Verfahren den Vorteil, dass man zur Gewinnung der R*,S*-Diastereomeren auf eine Trennung der Diastereomeren verzichten kann, was ja mit beträchtlichem Substanzverlust verbunden ist.

Beispiel 1

7,87 g (0,025 mol) 8-Phenoxy-4(1,2,4-triazolyl)-1)-2,2-dimethyl-octanon-3 in 130 ml Methylenchlorid werden bei −30°C mit 5,69 g (0,03 mol) Titantetrachlorid in 20 ml Methylenchlorid versetzt. Für 30 Min. rührt man die Mischung bei Raumtemperatur, kühlt wieder auf −30°C ab und versetzt tropfenweise mit 3,2 g (0,0125 mol) Tetra-n-butylammoniumborhydrid in 20 ml Methylenchlorid. Man rührt noch eine Stunde bei Raumtemperatur und versetzt dann langsam mit 200 ml

destilliertem Wasser, säuert mit 10%iger Säure deutlich an und trennt die Methylenchloridphase ab. Man schüttelt nochmals mit Methylenchlorid aus, wäscht mit verdünnter Salzsäure, Bicarbonatlösung und Wasser, trocknet und engt ein.

Man erhält 6,48 g (82% der berechneten Menge) festes Rohprodukt, das praktisch ausschliesslich das gewünschte Diastereomer R*,S* (9 $H_S$ bei 1,0 ppm) enthält. Nach einmaligem Umkristallisieren aus Toluol besitzt das Produkt einen Schmelzpunkt von 132 bis 133 °C.

Vergleich

Der Versuch des Beispiels 1 wird ohne $TiCl_4$-Zugabe durchgeführt und ergibt 6,2 g (78% der berechneten Menge) Rohprodukt, das nach dem NMR-Spektrum die beiden diastereomeren Carbinole R*,S* und R*,R* im Verhältnis 1:9 (9 $H_S$ bei 1,0 und 0,7 ppm) enthält, das sind 8% des gewünschten Produktes R*,S*.

Beispiel 2

Wie in Beispiel 1 beschrieben, wurden 6,43 g (0,025 mol) 5-Phenyl-4-(1,2,4-triazolyl-1)-2,2-dimethylpentanon-3 umgesetzt. Man erhielt 6,17 g (95% der Th.) Rohprodukt, das die beiden diastereomeren Carbinole R*,S* und R*,R* im Verhältnis 95:5 enthält (9 H-Singuletts bei 1,0 und 0,7 ppm im Verhältnis 95:5).

Beispiel 3

Wie in Beispiel 1 beschrieben, wurden 8,3 g (0,025 mol) 8-(o-Fluorphenoxy)-4-(1,2,4-triazolyl-1)-2,2-dimethyl-octanon-3 umgesetzt. Man erhielt 8,1 g (97% d. Th.) der diastereomeren Carbinole R*,S* und R*,R* im Verhältnis 95:5.

Beispiel 4

Wie in Beispiel 1 beschrieben, wurden 8,3 g (0,025 mol) 8-(o-Fluorphenoxy)-4(1,2,4-triazolyl-1)-2,2-dimethyl-octen-(6)-on-(3) umgesetzt. Man erhielt 8,2 g (99% d. Th.) Rohpodukt, das die diastereomeren Carbinole R*,S* und R*,R* wiederum im Verhältnis 95:5 enthält.

Beispiel 5

Wie in Beispiel 1 beschrieben, wurden 7,3 g (0,025 mol) 1-(p-Chlorphenoxy)-1-(1,2,4-triazolyl-1)-3,3-dimethyl-butanon-2 umgesetzt. Man erhält 7,2 g (98% d. Th.) Rohprodukt, das die beiden diastereomeren Carbinole R*,S* und R*,R* im Verhältnis 7:3 (9 H-Singuletts bei 1,1 und 0,8 ppm im Verhältnis 7:3) enthält.

Beispiel 6

8.2 g (0,025 mol) 1-(2,4-dichlorphenoxy)-1-(1,2,4-triazolyl-1)-3,3-dimethyl-butanon-2 werden entsprechend Beispiel 1 umgesetzt. Man erhält 8.1 g (98% d. Th.) Rohprodukt, das die beiden diastereomeren Carbinole R*,S* und R*,R* im Verhältnis 4:1 enthält (9 H-Singuletts bei 1,0 und 0,8 ppm im Verhältnis 4:1).

Beispiel 7

4,1 g (0,03 mol) im Hochvakuum getrocknetes Zinkchlorid werden in 4,5 g (0,06 mol) Diethylether (wasserfrei) gelöst und mit 100 ml Methylenchlorid verdünnt (klare Lösung). Zu dieser Lösung werden bei −30 °C 7,8 g (0,025 mol) 5-Phenyl-4-(1,2,4-triazolyl-1)-2,2-dimethyl-pentanon-3 in 40 ml $CH_2Cl_2$ zugegeben. Man rührt eine halbe Stunde bei Raumtemperatur, kühlt auf −30 °C ab und versetzt tropfenweise mit 3,2 g (0,0125 mol) Tetrabutylammoniumboranat in 20 ml Methylenchlorid. Es wird noch für 15 h bei Raumtemperatur nachgerührt und anschliessend wie unter Beispiel 1 beschrieben, aufgearbeitet. Man erhält 4,7 g (59% d. Th.) Rohprodukt, das die beiden diastereomeren Carbinole R*,S* und R*,R* im Verhältnis 9:1 (9 H-Singuletts bei 1,0 und 0,7 ppm im Verhältnis 9:1).

Beispiel 8

15,75 g (0,05 mol) 8-Phenoxy-4(1,2,4-triazolyl-1)-2,2-dimethyl-octanon-3, gelöst in 150 ml Methylenchlorid, werden bei −50 °C innerhalb 15 Min. mit 11,38 g (0,06 mol) Titantetrachlorid versetzt und für 30 Min. bei −50 °C nachgerührt. Anschliessend werden 50 ml THF zugegeben, auf 0 °C erwärmt und mit 1 g (0,026 mol) Lithiumaluminiumchlorid (Tablettenform) versetzt. Man lässt unter Rühren langsam auf Raumtemperatur kommen und rührt die Mischung für 70 h nach. Anschliessend wird auf 200 ml Eiswasser gegossen und mit 10%iger wässriger Salzsäure angesäuert. Die Methylenchloridphase wird abgetrennt, nochmals mit Methylenchlorid ausgeschüttelt, neutral gewaschen, getrocknet und eingeengt. Man erhält 14,26 g (90% d. Th.) festes Rohprodukt, das sich im NMR-Spektrum (200 MHz) als Gemisch aus

| | |
|---|---|
| Edukt | < 5% (9 $H_S$ 1,2 ppm) |
| R*,S*-Carbinol | ca. 72% (9 $H_S$ 1,0 ppm) |
| R*,R*-Carbinol | ca. 23% (9 $H_S$ 0,7 ppm) |

Einmaliges Umkristallisieren aus Toluol liefert 6,31 g (40% d. Th.) reines R*,S*-Carbinol, Fp. 132–133 °C.

Vergleichsversuch zu Beispiel 7

Verfährt man wie in Beispiel 7, aber ohne Titantetrachlorid-Zugabe, so erhält man 13.3 g (83% d. Th.) festes Rohprodukt, das sich im NMR-Spektrum (200 MHz) als Gemisch aus

| | |
|---|---|
| Edukt | < 2% |
| R*,S*-Carbinol | 56% (9 $H_S$ 1,0 ppm) |
| R*,R*-Carbinol | 44% (9 $H_S$ 0,7 ppm) |

erweist.

Beispiel 8

In 130 ml Methylenchlorid werden bei −30 °C 2,85 g (0,015 mol) Titantetrachlorid und 4,25 g (0,015 mol) Tetraisopropoxititanat zusammengegeben, auf Raumtemperatur erwärmt und für 0,5 h auf dieser Temperatur gehalten. Dabei bildet sich das Diisopropoxytitandichlorid. Nach dem Abkühlen auf −30 °C werden 7,87 g (0,025 mol) 8-Phenoxy-4(1,2,4-triazolyl-1)-2,2-dimethyl-octanon-3 in 40 ml Methylenchlorid zugegeben. Es wird eine halbe Stunde nachgerührt und dann bei 0 °C mit 3,2 g (0,0125 mol) Tetra-n-butylammoniumborhydrid in 20 ml Methylenchlorid tropfenweise versetzt. Nach dem Aufarbeiten erhält man 5,1 g (64% d. Th.) Rohprodukt, das die diastereomeren Carbinole R*,S* und R*,R* im Verhältnis 9:1 enthält (9 H-Singuletts bei 1,0 und 0,7 ppm im Verhältnis 9:1).

Beispiel 9

12,8 g (0,04 mol) 8-Phenoxy-4-(1,2,4-triazolyl-1)-2,2-dimethyl-octen-(6)-on-(3) in 50 ml Dichlormethan werden bei −30 °C mit 7,6 g (0,04 mol) Titantetrachlorid in 20 ml Dichlormethan versetzt. Sodann gibt man portionsweise eine Suspension von 1,8 g (0,02 mol) Tetramethylammoniumborhydrid in 20 ml Dichlormethan unter Kühlung zu, wobei die Temperatur bis auf −5 °C anstieg. Man belässt die Mischung eine Stunde bei Raumtemperatur und giesst sie sodann unter Kühlung auf eine Lösung von 12,8 g Natriumhydroxid in 200 ml Wasser, filtriert vom Unlöslichen ab, trennt die organische Phase des Filtrates, wäscht sie zweimal mit Wasser, trocknet sie über Magnesiumsulfat und engt i. Vak. zur Trockne ein. Der kristalline Rückstand (10,8 g, 84% d. Th.) enthält reines R*S*-8-Phenoxy-4-(1,2,4-triazolyl-1)-2,2-dimethylocten-(6)-ol-(3); der Anteil aus R*R*-Diastereomeren liegt unter 3% (1H-NMR).

Beispiel 10

9,82 g (0,025 mol) 6-(4-tert.-Butylphenoxy)-4-(1,2,4-triazolyl-1)-2,2-dimethylhexanon-(3) in 50 ml Dichlormethan werden bei −30 °C mit 5,7 g (0,03 mol) Titantetrachlorid in 20 ml Dichlormethan versetzt und für 0,5 Stunden gerührt. Anschliessend versetzt man mit 3,2 g (0,0125 mol) Tetrabutylammoniumborhydrid in 20 ml Dichlormethan. Die Temperatur steigt auf Raumtemperatur an, bei der für 6 Stunden nachgerührt wird. Nach Zugabe von 80 ml 10%iger HCl fallen 7,1 g (71% d. Th.) eines Festkörpers aus, der bei 157 bis 160 °C schmilzt.

$C_{20}H_{31}N_3O_2 \cdot HCl \cdot H_2O$ (398,5)
ber. C 60,07% H 8,51% N 10,50%
gef. C 59,4% H 8,6% N 10,0%

Das 1H-NMR-Spektrum ergibt einen R*S*-Anteil von 6-(4-tert.-Butylphenoxy)-4-(1,2,4-triazolyl-1)-2,2-dimethylhexanol-3 von über 97%.

Beispiel 11

Analog Beispiel 1 wurden 8,3 g (0,025 mol) 8-(0-Fluorphenoxy)-4-(1,2,4-triazolyl-1)-2,2-dimethyloctanon-3 umgesetzt, mit dem Unterschied, dass statt Titantetrachlorid 7,8 g (0,03 mol) Zinntetrachlorid verwendet wurden. Man erhält 7,9 g (94% d. Th.) festes Rohprodukt, das sich nach dem 1H-NMR-Spektrum wie folgt zusammensetzt: ca. 60% Ausgangsketon, 37% R*,S*-Carbinol, 3% R*,R*-Carbinol.

## Patentansprüche

1. Verfahren zur Herstellung von β-Triazolylcarbinolen in überwiegend R*,S*-diastereomerer Form

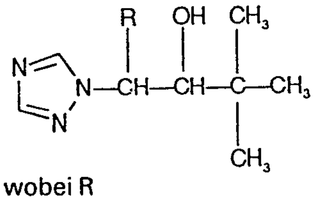

(I)

wobei R

a) Phenoxy, gegebenenfalls substituiert durch Halogen, $CF_3$, Alkyl, Alkoxy, Phenyl, Phenoxy oder

b) $C_1$-$C_{10}$-Alkyl, gegebenenfalls ein- oder mehrfach substituiert durch gleiche oder verschiedene Reste aus den Gruppen Alkoxy, Alkyl, Aryl, Aryloxy, die ihrerseits wiederum durch Halogen, $CF_3$, Alkyl oder Alkoxy substituiert sein können, bedeutet,

durch diastereoselektive Reduktion eines entsprechenden α-Triazolylketons mit einem komplexen, Ketone zu Alkoholen reduzierenden Hydrid dadurch gekennzeichnet, dass man die Umsetzung in Gegenwart einer Lewis-Säure in einem aprotischen Lösungsmittel vornimmt.

2. Verfahren nach Anspruch 1, dadurch gekennzeichnet, dass man als komplexes Hydrid ein Tetraalkylammoniumborhydrid oder ein in aprotischen Lösungsmitteln lösliches Metallhydrid verwendet.

3. Verfahren nach Anspruch 1, dadurch gekennzeichnet, dass man als Lewis-Säure Titantetrachlorid, Zinkchlorid oder Zinntetrachlorid verwendet.

## Claims

1. A process for the preparation of a β-triazolyl-cararbinol in a predominantly R*,S*-diastereomeric form

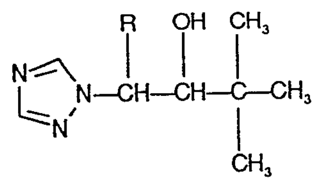

(I)

where R is

a) phenoxy which is unsubstituted or substituted by halogen, $CF_3$, alkyl, alkoxy, phenyl or phenoxy, or

b) $C_1$-$C_{10}$-alkyl which is unsubstituted or monosubstituted or polysubstituted by identical or different radicals from the group consisting of alkoxy, alkyl, aryl and aryloxy, which in turn can be substituted by halogen, $CF_3$, alkyl or alkoxy, by diastereoselective reduction of a corresponding α-triazolylketone with a complex hydride which reduces ketones to alcohols, wherein the reaction is carried out in the presence of a Lewis acid in an aprotic solvent.

2. A process as claimed in claim 1, wherein the complex hydride used is a tetraalkylammonium borohydride, or a metal hydride which is soluble in aprotic solvents.

3. A process as claimed in claim 1, wherein the Lewis acid used is titanium tetrachloride, zinc chloride or tin tetrachloride.

**Revendications**

1. Procédé de préparation de β-triazolylcarbinoles essentiellement sous forme R*,S* diastereomère

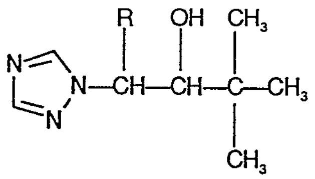

(I)

R représentant

a) phénoxy, éventuellement substitué par halogène, $CF_3$, alkyle, alcoxy, phényle, phénoxy ou

b) alkyle en $C_1$–$C_{10}$, éventuellement substitué une ou plusieurs fois par des restes identiques ou différents des groupes alcoxy, alkyle, aryle, aryloxy, qui peuvent, à leur tour, être à nouveau substitué par halogène, $CF_3$, alkyle ou alcoxy par réduction diastéréosélective d'une α-triazolylcétone correspondante avec un hydrure complexe, réduisant les cétones en alcools, caractérisé par le fait qu'un effectue la réaction dans un solvant aprotique en présence d'un acide de Lewis.

2. Procédé selon la revendication 1, caractérisé par le fait qu'on utilise comme hydrure complexe un borohydrure de tétralkylammonium ou un hydrure métallique soluble dans les solvants aprotiques.

3. Procédé selon la revendication 1, caractérisé par le fait qu'on utilise, comme acide de Lewis, du tétrachlorure de titane, du chlorure de zinc ou du tétrachlorure d'étain.